# EUROPÄISCHE PATENTSCHRIFT

(11) **EP 0 371 921 B1**
(45) Veröffentlichungstag und Bekanntmachung des Hinweises auf die Patenterteilung: **08.06.1994**
(21) Anmeldenummer: 89810884.0
(22) Anmeldetag: 20.11.1989
(51) Int. Cl.: C07F 9/38, A61K 31/66

(54) **Phenylaliphatylaminoalkandi-phosphonsäuren**
Phenyl aliphatyl amino alkane diphosphonic acids
Acides phénylaliphatylaminoalcane diphosphoniques

(30) Priorität: 28.11.1988 CH 4404/88
(43) Veröffentlichungstag der Anmeldung: 06.06.1990
(73) Patentinhaber: CIBA-GEIGY AG, 4002 Basel (CH)
(72) Erfinder: Jaeggi, Knut A., Dr., CH-4054 Basel (CH)

(56) Entgegenhaltungen:
- EP-A- 0 252 504
- EP-A- 0 320 455

## Beschreibung

Die Erfindung betrifft neue Aminoalkandiphosphonsäurederivate zur Behandlung von störungen des Calciumstoffwechsels.

In EP-A-252504 werden u.a. die 3-(N-Benzyl-N-methylamino)propan-1-hydroxy-1,1-diphosphonsäure und diese enthaltende Arzneimittel offenbart.

Aufgabe der Erfindung dem gegenüber ist es, Produkte mit verbesserten pharmakologischen Eigenschaften bereitzustellen.

Gegenstand der Erfindung sind dem nach aromatisch substituierte Alkylaminoalkandiphosphonsäuren der Formel 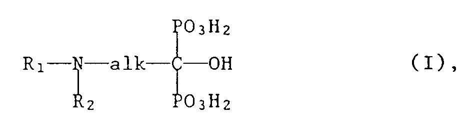
worin R₁ Phenyl-C₄-C₇-alkyl oder Phenyl-C₄-C₇-alkenyl bedeutet, R₂ C₁-C₄-Alkyl oder C₂-C₄-Alkenyl darstellt und alk für C₂-C₄-Alkylen steht, und ihre Salze, Verfahren zur Herstellung der erfindungsgemässen Verbindungen, diese enthaltende pharmazeutische Präparate und ihre Verwendung zur Herstellung von Arzneimittel.

Nachstehend sind unter niederen Resten und Verbindungen beispielsweise solche zu verstehen, die bis und mit 7, insbesondere bis und mit 4, C-Atome aufweisen. Ferner haben die Allgemeinbegriffe beispielsweise folgende Bedeutungen:
C₁-C₄-Alkyl ist insbesondere Methyl oder in zweiter Linie Aethyl, Propyl, Isopropyl oder Butyl, ferner Iso- oder Sekundärbutyl.

Phenyl-C₄-C₇-alkyl ist beispielsweise geradkettiges Phenyl-C₄-C₇-alkylen, vor allem ω-Phenyl-C₄-C₆-alk-1-yl, wie 4-Phenylbut-1-yl, 5-Phenylpent-1-yl oder 6-Phenylhex-1-yl, ferner 3-Phenyl-but-1-yl oder 4-Phenylpent-1-yl.

Phenyl-C₄-C₇-alkenyl ist beispielsweise geradkettiges Phenyl-C₄-C₇-alkenyl, vor allem solches, in dem sich die Doppelbindung in höherer als der α,β-Stellung zum N-Atom befindet, wie 4-Phenylbut-3-en-1-yl, 4-Phenylbut-2-en-1-yl oder 5-Phenylpent-4-en-1-yl.

C₂-C₄-Alkenyl ist beispielsweise Vinyl, Allyl oder Buten-2-yl.

C₂-C₄-Alkylen ist beispielsweise geradkettiges C₂-C₄-Alkylen, vor allem geradkettiges C₂-C₃-Alkylen, wie α,ω-C₂-C₄-Alkylen, z.B.Aethylen, 1,3-Propylen oder in zweiter Linie 1,4-Butylen.

Salze von Verbindungen der Formel I sind insbesondere deren innere Salze oder Salze mit pharmazeutisch verwendbaren Basen, wie nicht-toxische, von Metallen oder Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, Kupfer-, Aluminium- oder Zinksalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di-oder Triniederalkylaminen, z.B. Methyl-, Aethyl-, Dimethyl- oder Diäthylamin, Mono-, Di- oder Tri-(hydroxyniederalkyl)-aminen, wie Aethanol-, Diäthanol- oder Triäthanolamin, Tris(hydroxymethyl)-amino-methan oder 2-Hydroxytertiärbutylamin, oder N-(Hydroxyniederalkyl)-N,N-diniederalkylaminen bzw. N-(Polyhydroxyniederalkyl)-N-niederalkylaminen, wie 2-(Dimethylamino)äthanol oder D-Glucamin, oder quaternären aliphatischen Ammoniumhydroxiden, z.B. mit Tetrabutylammoniumhydroxid. Umfasst sind sowohl vollständige als auch partielle Salze, d.h. Salze mit 1,2,3 oder 4, vorzugsweise 2, Aequivalenten Base pro Mol der Säure der Formel I.

Die Verbindungen der Formel I und ihre Salze weisen wertvolle pharmakologische Eigenschaften auf. Insbesondere besitzen sie eine ausgeprägte regulierende Wirkung auf den Calcium-Stoffwechsel von Warmblütern.

Insbesondere bewirken sie an der Ratte eine ausgeprägte Hemmung der Knochenresorption, die sich sowohl in der Versuchsanordnung gemäss Acta Endocrinol. 78, 613-24 (1975) anhand des PTH-induzierten Anstieges des Serumcalciumspiegels nach subcutaner Applikation in Dosen von etwa 0,01 bis etwa 1,0 mg/kg, als auch im TPTX (Thyroparathyroidectomised) -Rattenmodell anhand der durch Vitamin D₃ ausgelösten experimentellen Hypercalcämie nach Gabe von Dosen von etwa 0,001 bis 0.01 mg s.c. zeigen lässt. Ebenso wird die durch Walker-256-Tumore induzierte Tumorhyperkalzämie nach peroraler Verabreichung von etwa 1,0 bis etwa 100 mg/kg gehemmt. Ferner zeigen sie in der Adjuvansarthritis der Ratte in der Versuchsanordnung nach Newbould, Brit. J. Pharmacology 21, 127 (1963) sowie nach Kaibara et al., J. Exp. Med. 159, 1388-96 (1984) in Dosen von etwa 0,01 bis 1,0 mg/kg s.c. eine deutliche Hemmung auf das Fortschreiten chronisch-arthritischer Prozesse. Sie sind deshalb vorzüglich geeignet als Arzneimittelwirkstoffe für die Behandlung von Erkrankungen, die mit Störungen des Calciumstoffwechsels in Verbindung gebracht werden können, beispielsweise entzündlicher Prozesse in Gelenken, degenerativer Prozesse im Gelenkknorpel, von Osteoporosis, Periodontitis, Hyperparathyreoidismus und von Calciumablagerungen in Blutgefässen oder an prothetischen Implantaten. Günstig beeinflusst werden sowohl Erkrankungen, bei denen eine anomale Ablagerung schwerlöslicher Calciumsalze festzustellen ist, wie solcher aus den Formenkreisen der Arthritis, z.B. Morbus Bechterew, der Neuritis, der Bursitis, Periodontitis und der Tendinitis, der Fibrodysplasie, der Osteoarthrose oder der Artereosklerose, als auch solche, bei denen eine anomale Auflösung harter Körpergewebe im Vordergrund steht, wie die hereditäre Hypophosphatasie, degenerative Prozesse im Gelenkknorpel, Osteoporosen verschiedener Genese, morbus Paget und die Osteodystrophia fibrosa, ebenso durch Tumore ausgelöste osteolytische Prozesse.

Die Erfindung betrifft ganz bevorzugt Verbindungen der Formel I, worin R₁ geradkettiges Phenyl-C₄-C₇-alkyl, wie 4-Phenylbut-1-yl, 5-Phenylpent-1-yl oder 6-Phenylhex-1-yl, bedeutet, R₂ C₁-C₄-Alkyl, wie Methyl, darstellt und alk C₂-C₃-Alkylen, wie Aethylen, bedeutet, und ihre Salze, insbesondere pharmazeutisch verwendbaren Salze.

Die Erfindung betrifft in allererster Linie Verbindungen der Formel I, worin R₁ geradkettiges ω-Phenyl-C₄-C₆-alk-1-yl, wie 4-Phenylbut-1-yl oder 5-Phenylpent-1-yl, steht, alk' geradkettiges C₂-C₄-Alkylen bedeutet, R₂ für C₁-C₄-Alkyl steht und alk C₂-C₃-Alkylen, wie Aethylen, darstellt, und ihre Salze, insbesondere pharmazeutisch verwendbaren Salze.

Die Erfindung betrifft namentlich die in den Beispielen genannten Verbindungen der Formel I und ihre Salze, insbesondere ihre inneren Salze und pharmazeutisch verwendbaren Salze mit Basen.

Die Erfindung betrifft ferner ein auf an sich bekannten Methoden beruhendes Verfahren zur Herstellung von Verbindungen der Formel I und ihrer Salze. Dieses ist dadurch gekennzeichnet, dass man
a) in einer Verbindung der Formel 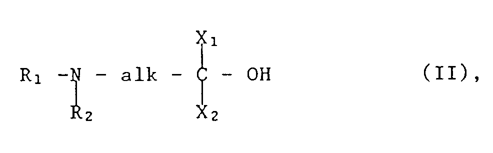 worin X₁ eine funktionell abgewandelte und X₂ eine freie oder funktionell abgewandelte Phosphonogruppe bedeutet, funktionell abgewandeltes Phosphono X₁ und gegebenenfalls X₂ in die freie Phosphonogruppe überführt, oder
b) Verbindungen der Formeln

   R_{A} - Y₁ (III)

   und 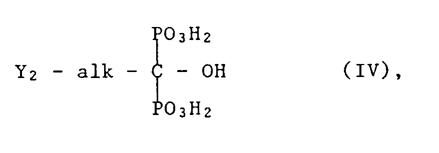 worin einer der Reste Y₁ und Y₂ eine reaktionsfähige veresterte Hydroxygruppe und der andere eine Gruppe der Formel -N(R_{B})-H bedeutet, wobei einer der Reste R_{A} und R_{B} einen Rest R₁ und der andere einen Rest R₂ darstellt, oder deren Salze, miteinander umsetzt, oder unter reduzierenden Bedingungen Verbindungen der Formeln

   R_{C}=O (IIIa)

   und 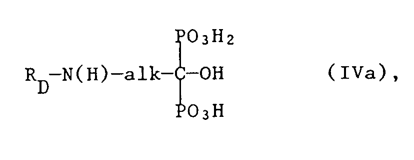 worin R_{C} ein dem Rest R₁ entsprechender doppelt gebundener Rest, z.B. Phenyl-C₄-C₇-alkylidenrest oder Phenyl-C₄-C₇-alkenylidenrest, und R_{D} einen Rest R₂ oder R_{C} einen dem Rest R₂ entsprechender doppelt gebundener Rest, z.B. C₁-C₄-Alkyliden, und R_{D} einen Rest R₁ bedeutet, oder
c) eine Verbindung der Formel 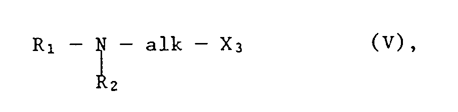 worin X₃ Carboxy, Carbamyl oder Cyano bedeutet, mit einem Phosphorylierungsmittel umsetzt und das Primärprodukt hydrolysiert; und im Falle von X₃=Cyano oder X₃=Carbamyl das erhaltene Zwischenprodukt der Formel 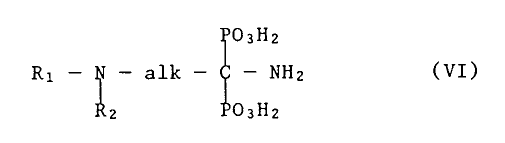 bzw. eine Salz davon mit salpetriger Säure behandelt, und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

Gemäss der Verfahrensvariante a) in Phosphono zu überführende funktionell abgewandelte Phosphonogruppen liegen beispielsweise in einer Esterform, insbesondere einer Diesterform der Formel -P(=O)(OR)₂ (IIa), vor, worin OR veräthertes Hydroxy, beispielsweise Niederalkoxy, Niederalkanoyloxyniederalkoxy, wie C₂-C₇-Alkanoyloxy-C₁-C₄-alkoxy, z.B. Acetyl-oder Pivaloyloxymethoxy, oder eine gegebenenfalls durch Niederalkyl, Niederalkoxy, Halogen, Trifluormethyl und/oder Hydroxy substituierte Phenyloxy- oder α-Phenylniederalkoxygruppe oder Silyloxy, wie Triniederalkylsilyloxy, bedeutet.

Die Ueberführung von funktionell abgewandelten in freie Phosphonogruppen erfolgt in üblicher Weise, wie durch Hydrolyse, beispielsweise in Gegenwart einer Mineralsäure, wie Salz- oder Schwefelsäure, bei etwa 80°C bis etwa 110°C, z.B. in der Siedehitze, oder durch Umsetzung mit einem Triniederalkyl-halogensilan, z.B. mit Trimethyl-chlorsilan oder insbesondere Trimethyl-jodsilan oder Trimethyl-bromsilan, vorzugsweise in Methylenchlorid im Temperaturbereich von etwa 0° bis etwa 40°C, und anschliessende Behandlung mit Wasser. α-Phenylniederalkylester können ferner durch Hydrogenolyse, beispielsweise Umsetzung mit Wasserstoff in Gegenwart eines Hydrierungskatalysators, wie eines Nickel- oder Edelmetallkatalysators, z.B. von Palladium auf Kohle, vorzugsweise in einem Niederalkanol unter normalen Druck- und Temperaturbedingungen in Verbindungen der Formel I überführt werden.

Die Ausgangsstoffe der Formel II können beispielsweise hergestellt werden, indem man eine Verbindung der Formel 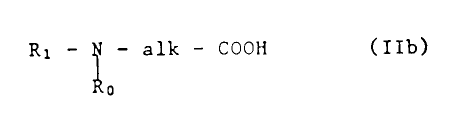
worin R₀ einen Rest R₂ oder eine Aminoschutzgruppe darstellt, oder vorzugsweise das Anhydrid oder Säurechlorid derselben mit einem entsprechenden Phosphorigsäuretriester der Formel P(OR)₃ (IIc), beispielsweise bei 0°C bis etwa 60°C, zu einer Verbindung der Formel 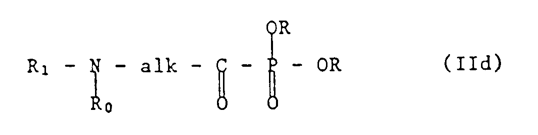
und diese mit einem Phosphorigsäurediester der Formel H-P(=O)(OR)₂ (IIe) bzw. P(OH)(OR)₂ (IIf) in Gegenwart eines Diniederalkylamins, z.B. von Diäthylamin, oder eines Alkalimetallniederalkanolats, z.B. Natriummethylat, zur entsprechenden Verbindung der Formel 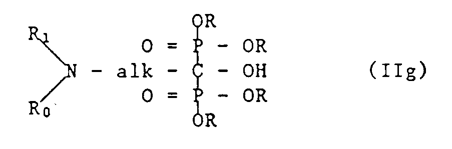
weiterumsetzt, gegebenenfalls die Aminoschutzgruppe abspaltet und den Rest R₂ einführt, z.B. wie nachstehend unter b) beschrieben. Ausgangsstoffe der Formel IIb können, soweit sie nicht bekannt sind, beispielsweise hergestellt werden, indem man eine entsprechende Verbindung der Formel

R₁-N(R₀)-H (IIh),

worin R₀ eine Gruppe R₂ oder eine Aminoschutzgruppe bedeutet, mit einer Verbindung der Formel

Y - alk - COOR (IIi),

worin Y Halogen, wie Brom ist, oder zur Herstellung von Verbindungen IIb, worin alk 1,2-Niederalkylen, z.B. Aethylen, bedeutet, einer Verbindung der Formel

alk₀ - COOR (IIj)

worin alk₀ Niederalk-1-enyl bedeutet, umsetzt, jeweils den erhaltenen Ester zur Säure hydrolysiert, diese, z.B. mittels Phosphorpentachlorid, anhydridisiert bzw. chloriert und gewünschtenfalls die Aminoschutzgruppe, sofern vorhanden, abspaltet.

Gemäss der Verfahrensvariante b) zu verwendende reaktive Ester (III) bzw. (IV) weisen als reaktionsfähige veresterte Hydroxygruppe beispielsweise ein Halogen-, wie Chlor-, Brom- oder Jodatom oder eine Sulfonyloxygruppe, wie Alkan- oder gegebenenfalls substituiertes Benzolsulfonyloxy, z.B. Methan- oder p-Toluolsulfonyloxy, auf.

Die Umsetzung mit den genannten reaktiven Estern erfolgt beispielsweise in Gegenwart einer Base, wie eines Alkalimetall- oder Erdalkalimetallhydroxides, z.B. von Natriumhydroxid, oder eines quaternären Ammoniumhydroxides, z.B. von Tetrabutylammoniumhydroxid, vorteilhaft in Gegenwart eines Lösungs- oder Verdünnungsmittels, z.B. eines Niederalkanols, Diniederalkylketons oder cycloaliphatischen Aethers, z.B. von Isopropanol, Methyläthylketon, Dioxan oder Tetrahydrofuran.

Die Umsetzung mit Oxoverbindungen IIIa wird beispielsweise in Gegenwart eines geeigneten Reduktionsmittel, wie eines Alkalimetallborhybrides, z.B. von Natriumcyanoborhybrid, oder insbesondere von Ameisensäure durchgeführt. Insbesondere kann man eine Verbindung der Formel IVa, worin R_{D} einen Rest R₁ bedeutet unter reduzierenden Bedingungen mit einem Niederalkanal, beispielsweise mit Formaldehyd, und Ameisensäure zur entsprechenden Verbindung der Formel I umsetzen, worin R₂ Niederalkyl, beispielsweise Methyl, bedeutet. Alternativ kann man auch durch Umsetzung mit einem reaktionsfähigen Ester eines Niederalkanols bzw. Niederalkenols in üblicher Weise, vorzugsweise in Gegenwart eines basischen Kondensationsmittels, wie eines Alkalimetallniederalkanolates, Niederalkyl bzw. Niederalkenyl R₂ einführen.

Die Ausgangsstoffe der Formel IV können beispielsweise hergestellt werden, indem man eine Verbindung der Formel

Y₂ - alk - COOH (IVa)

in üblicher Weise, beispielsweise in Chlorbenzol, mit phosphoriger Säure und Phosphortrichlorid bzw. mit Phosphorsäure und einem Ueberschuss an Phosphortribromid umsetzt und anschliessend hydrolytisch aufarbeitet.

Als Phosphorylierungsmittel für Verfahrensvariante c) kommen beispielsweise Phosphortrioxid, Phosphortrihalogenide im Gemisch mit phosphoriger Säure oder Phosphorsäure, Phosphoroxychlorid, Phosphorpentachlorid bzw. Phosphortrichlorid und Chlor in Betracht. Bevorzugt ist Phosphortrioxid, welches vorzugsweise durch Umsetzung von Phosphortrichlorid mit phosphoriger Säure bzw. durch Reaktion überschüssigen Phosphortrichlorides mit wasserhaltiger Phosphorsäure, z.B. mit handelsüblicher etwa 75%-iger bis etwa 95%-iger, vorzugsweise etwa 85%-iger Phosphorsäure, in situ gebildet wird. Die Umsetzung wird vorteilhaft unter Erwärmen, z.B. auf etwa 70° bis etwa 120°C, in einem geeigneten Lösungsmittel, wie Tetrachloräthan, Trichloräthan, Chlorbenzol, Chlortoluol oder Paraffinöl, und unter hydrolytischer Aufarbeitung durchgeführt.

Die Behandlung von Zwischenprodukten der Formel VI mit salpetriger Säure erfolgt in üblicher Weise unter Freisetzung derselben in wässriger Lösung aus einem ihrer Salze, z.B. aus Natriumnitrit, durch Säurebehandlung, z.B. Einwirkung von Salzsäure, wobei intermediär ein entsprechendes, instabiles Diazoniumsalz, z.B. -chlorid, gebildet wird, das unter Einführung der α-Hydroxygruppe Stickstoff abspaltet.

Die Ausgangsstoffe der Formel V können, soweit sie nicht bekannt sind, beispielsweise hergestellt werden, indem man eine entsprechende Verbindung der Formel

R₁-N(R₂)-H (IIh)

Verbindung der Formel

Y - alk - X₃ (IIi),

worin Y Halogen, wie Brom ist, oder zur Herstellung von Verbindungen der Formel V, worin alk 1,2-Niederalkylen, z.B. Aethylen, bedeutet, mit einer Verbindung der Formel

alk₀ - X₃ (IIf)

worin alk₀ einen Niederalk-1-enylrest bedeutet, umsetzt, jeweils die Aminoschutzgruppe, sofern vorhanden, abspaltet und gewünschtenfalls jeweils das erhaltene Primärprodukt zur Säure hydrolysiert.

Verfahrensgemäss oder nach einem anderen an sich bekannten Verfahren erhaltene Verbindungen der Formel I können in an sich bekannter Weise in andere Verbindungen der Formel I überführt werden.

So kann man in R₁ und/oder R₂ vorhandene nicht-aromatische Doppelbindungen in üblicher Weise durch Hydrogenolyse, beispielsweise Umsetzung mit Wasserstoff in Gegenwart eines Hydrierungskatalysators, wie eines Nickel-oder Edelmetallkatalysators, z.B. von Palladium auf Kohle, vorzugsweise in einem Niederalkanol unter normalen Druck- und Temperaturbedingungen, zu Einfachbindungen reduzieren.

Die neuen Verbindungen können, je nach der Wahl der Ausgangsstoffe und Arbeitsweisen, in Form eines der möglichen Isomeren oder als Gemisch derselben, z.B. je nach der Anzahl der asymmetrischen Kohlenstoffatome als reine optische Isomere, wie Antipoden oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen, oder durch Umsetzung eines sauren Endstoffes mit einer mit der racemischen Säure Salze bildenden optisch aktiven Base und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können, zerlegen. Vorteilhaft isoliert man den wirksameren der beiden Antipoden.

Erhaltene freie Verbindungen der Formel I einschliesslich ihrer inneren Salze der Formel I können durch partielle oder vollständige Neutralisation mit einer der eingangs genannten Basen in Basesalze überführt werden. In analoger Weise kann man auch Säureadditionssalze in die entsprechenden freien Verbindungen deren innerer Salze überführen.

Umgekehrt kann man erhaltene freie Verbindungen der Formel I durch Behandlung mit einer Protonensäuren in Säureadditionssalze überführen.

Erhaltene Salze können in an sich bekannter Weise in andere Salze mit geringerem Kationenanteil (partielle Salze) oder in die freien Verbindungen umgewandelt werden, z.B. durch Behandeln mit einem sauren Reagens, wie einer Mineralsäure. Erhaltene freie Verbindungen können durch Behandlung mit einer Base, z.B. Alkalilauge in Salze und/oder erhaltene Salze mit geringerem Kationenanteil (partielle Salze) auf die gleiche Weise in solche mit höherem Kationenanteil, z.B. vollständige Salze, umgewandelt werden.

Die Verbindungen einschliesslich ihrer Salze können auch in Form ihrer Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu deren Herstellung bilden ebenfalls einen Gegenstand der Erfindung.

Bei den erfindungsgemässen pharmazeutischen Präparaten, welche Verbindungen der Formel I oder pharmazeutisch verwendbare Salze davon enthalten, handelt es sich um solche zur enteralen, wie oralen oder rektalen, und parenteralen Verabreichung, welche den pharmakologischen Wirkstoff allein oder zusammen mit einem pharmazeutisch anwendbaren Trägermaterial enthalten.

Die neuen pharmazeutischen Präparate enthalten z.B. von etwa 10 % bis etwa 80 %, vorzugseise von etwa 20 % bis etwa 60 % des Wirkstoffs. Erfindungsgemässe pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit und Cellulosepräparate ferner Bindemittel, wie Stärkekleister unter Verwendung z.B. von Mais-, Weizen-, Reis- oder Kartoffelstärke, Gelatine, Tragaganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthyleglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Die vorliegende Erfindung betrifft ebenfalls die Verwendung der Verbindungen der Formeln I und ihrer Salze, vorzugsweise zur Herstellung von Arzneimitteln zur Behandlung von auf Störungen des Calciumstoffwechsels zurückzuführenden Erkrankungen, z.B. des rheumatischen Formenkreises, und besonders von Osteoporosen.

Dosierungen unter 0,01 mg/kg Körpergewicht beeinflussen die pathologische Verkalkung bzw. die Auflösung von harten Geweben nur unerheblich. Bei Dosierungen von über 100 mg/kg Körpergewicht können langfristig toxische Nebenwirkungen auftreten. Die Verbindungen der Formel I und ihre Salze können sowohl oral als auch in hypertonischer Lösung subkutan, intramuskulär oder intravenös appliziert werden. Die bevorzugten Tagesdosen für diese Anwendungen liegen bei oraler Anwendung im Bereich von etwa 0,1 bis 5 mg/kg, bei subkutaner und intramuskulärer Applikation im Bereich von etwa 0,1 bis 1 mg/kg und bei intravenöser Applikation im Bereich von etwa 0,01 bis 2 mg/kg, beispielsweise von etwa 0,013 bis 0,67 mg/kg.

Die Dosierung der verwendeten Verbindungen ist jedoch variabel und hängt von den jeweiligen Konditionen wie Art und Schwere der Erkrankung, Dauer der Behandlung und der jeweiligen Verbindung ab. Einzeldosen enthalten beispielsweise von 0,01 bis 10 mg, Dosiseinheitsformen für parenterale, wie intravenöse Applikation z.B. von 0,01 bis 0,1 mg, vorzugsweise 0,02 bis 0,08 mg, orale Dosiseinheitsformen z.B. von 0,2 bis 2,5 mg, vorzugsweise 0,3 bis 1,5 mg pro kg Körpergewicht. Die bevorzugte Einzeldosierung beträgt bei oraler Applikation 10 bis 100 mg und bei intravenöser Applikation 0,5 bis 5 mg und kann bis zu 4-mal täglich verabreicht werden. Die höheren Dosierungen bei oraler Applikation sind infolge der begrenzten Resorption erforderlich. Bei längerdauernden Behandlungen kann nach anfänglich höherer Dosierung normalerweise auf niedrige Dosierungen umgestellt werden, um den gewünschten Effekt aufrechtzuerhalten.

Beispiel 1: 12,75 g (0,0446 Mol) 3-[N-(5-Phenylpentyl)-N-methyl-amino]-propionsäure-hydrochlorid werden mit 6,1 ml 85%-iger Phosphorsäure und 30 ml Chlorbenzol unter Rühren und Rückfluss auf 100° erhitzt. Dann werden bei 100° 11,7 ml Phosphortrichlorid zugetropft, wobei Gasentwicklung stattfindet. Das Reaktionsgemisch scheidet im Lauf von 30 Minuten eine dicke Masse ab. Man erhitzt noch 3 Stunden auf 100° und dekantiert dann das überstehende Chlorbenzol ab. Die zurückbleibende, zähe Masse wird mit 45 ml 9-n. Chlorwasserstoffsäure 3 Stunden unter Rühren und Rückfluss zum Sieden erhitzt. Man filtriert heiss unter Kohlezusatz und engt das Filtrat unter vermindertem Druck ein. Auf Zusatz von Aceton erhält man die rohe 3-[N-(5-Phenylpentyl)-N-methyl-amino]-1-hydroxy-propan-1,1-diphosphonsäure, welche aus Wasser umkristallisiert wird: Smp. 124-127° (Zers.).

Durch Lösen von 0,005 Mol derselben in 10 ml n-Natronlauge, Einengen unter vermindertem Druck und Kristallisieren unter Zugabe von Methanol erhält man das Dinatrium-3-[N-(5-Phenylpentyl)-N-methyl-amino]-1-hydroxy-propan-1,1-diphosphonat.

Das als Ausgangsmaterial dienende 3-[N-(5-Phenylpentyl)-N-methyl-amino]-propionsäure-hydrochlorid kann folgendermassen hergestellt werden:
10,0 g (0,056 Mol) N-(5-Phenylpentyl)-N-methyl-amin werden in 40 ml Diethyläther gelöst und mit 6,7 g Acrylsäureäthylester versetzt. Nach viertätigem Stehen bei Raumtemperatur wird der Aether abdestilliert. Das zurückbleibende Oel stellt den rohen 3-[N-(5-Phenylpentyl)-N-methyl-amino]-propionsäureäthylester dar.

14,2 g (0,05 Mol) des wie vorstehend erhaltenen Esters werden mit 80 ml 4-n. Chlorwasserstoffsäure 24 Stunden zum Rückfluss erhitzt. Dann wird unter vermindertem Druck völlig eingedampft und der kristalline Rückstand mit Aceton verrieben. Nach Abnutschen, Waschen und Trocknen der Kristalle erhält man das 3-[N-(5-Phenylpentyl)-N-methyl-amino]-propionsäure-hydrochlorid vom Smp. 97-99°.

Beispiel 2: 0,9 g (2,45 mMol) 3-(4-Phenylbutylamino)-1-hydroxy-propan-1,1-diphosphonsäure werden mit 7,5 ml 98%-iger Ameisensäure und 0,5 ml 35%-iger wässriger Formaldehyd-Lösung 20 Stunden unter Rühren und Rückfluss zum Sieden erhitzt.

Das Reaktionsgemisch wird unter vermindertem Druck eingeengt und der Rückstand aus Methanol kristallisiert. Man erhält die 3-[N-(4-Phenylbutyl)-N-methyl-amino]-1-hydroxy-propan-1,1-diphosphonsäure, Smp. 128-132° (Zers.).

Das Ausgangsmaterial kann z.B. folgendermassen hergestellt werden:
Ausgehend von N-(4-phenylbutyl)-N-benzyl-amin und Acrylsäureäthylester erhält man analog Beispiel 1 den 3-[N-(4-Phenylbutyl)-N-benzyl-amino]-propionsäure-äthylester, welcher mit Chlorwasserstoffsäure zum 3-[N-(4-Phenylbutyl)-N-benzyl-amino]-propionsäure-hydrochlorid, Smp. 145-147°, hydrolysiert wird.

27,13 g (0,085 Mol) dieses Hydrochlorids werden in 300 ml Aethanol über 3 g 5%-Palladium-auf-Kohle-Katalysator bei 20-25° und Normaldruck bis zum Ende der Wasserstoffaufnahme hydriert. Der Katalysator wird abgenuscht, das Filtrat eingedampft und der Rückstand aus Aceton kristallisiert. Man erhält das 3-[N-(4-Phenylbutyl)amino]propionsäure-hydrochlorid, Smp. 135-137°.

Analog Beispiel 1 erhält man aus 3-N-(4-Phenylbutyl-amino)propionsäure-hydrochlorid die 3-(4-Phenylbutylamino)-1,1-hydroxy-propan-1,1-diphosphonsäure, Smp. 191-193° (Zers.).

Beispiel 3: In analoger Weise wie in Beispiel 2 beschrieben kann man über 3-[N-(6-Phenylhexyl)amino]-propionsäure-hydrochlorid, Smp. 128-129°, und 3-N-(3-Phenylhexyl)amino]-1-hydroxy-propan-1,1-diphosphonsäure, Smp. 203-205° (Zers.) die 3-[N-(6-Phenylhexyl)-N-methyl-amino]-1-hydroxy-propan-1,1-diphosphonsäure, Smp. 149-152° (Zers.), herstellen.

Beispiel 4: In analoger Weise wie in Beispiel 1 beschrieben kann man über 3-[N-(7-Phenylheptyl)-N-methylamino]-propionsäure-hydrochlorid, Smp. 95-97°, 3-[N-(7-Phenylheptyl)-N-methyl-amino]-1-hydroxy-propan-1,1-diphosphonsäure, Smp. 138-142° (Zers.), herstellen.

Beispiel 5: Tabletten, enthaltend 75 mg Wirkstoff, z.B. 3-[N-(5-Phenylpentyl)-N-methyl-amino]-1-hydroxy-propan-1,1-diphosphonsäure oder ein Salz, z.B. das Dinatriumsalz, davon, können folgendermassen hergestellt werden:

| Bestandteile (für 1000 Tabletten) | |
|---|---|
| Wirkstoff | 75,0 g |
| Lactose | 268,5 g |
| Maisstärke | 22,5 g |
| Polyäthylenglykol 6000 | 5,0 g |
| Talkum | 15,0 g |
| Magnesiumstearat | 4,0 g |
| entmineralisiertes Wasser | q.s. |

Herstellung: Die festen Ingredienzien werden zunächst durch ein Sieb mit 0,6 mm Maschenweite getrieben. Dann wird der Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

Beispiel 6: Tabletten, enthaltend 10 mg Wirkstoff, z.B. 3-[N-(5-Phenylpentyl)-N-methyl-amino]-1-hydroxy-propan-1,1-diphosphonsäure oder ein Salz, z.B. das Dinatriumsalz davon, können folgendermassen hergestellt werden:

| Zusammensetzung (für 1000 Tabletten) | |
|---|---|
| Wirkstoff | 10,0 g |
| Lactose | 328,5 g |
| Maisstärke | 17,5 g |
| Polyäthylenglykol 6000 | 5,0 g |
| Talkum | 25,0 g |
| Magnesiumstearat | 4,0 g |
| entmineralisiertes Wasser | q.s. |

Herstellung: Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

Beispiel 7: Gelatinesteckkapseln, enthaltend 100 mg Wirkstoff, z.B. 3-[N-(5-Phenylpentyl)-N-methyl-amino]-1-hydroxy-propan-1,1-diphosphonsäure oder ein Salz, z.B. das Dinatriumsalz davon, können folgendermassen hergestellt werden:

| Zusammensetzung (für 1000 Kapseln) | |
|---|---|
| Wirkstoff | 350,0 g |
| microkristalline Cellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem Wirkstoff (lyophilisiert) gesiebt und beide Komponenten 10 Minuten lang innig vermischt. Dann wird die mikrokristalline Cellulose durch ein Sieb mit einer Maschenweite von 0,9 mm hinzugesiebt und erneut 10 Minuten innig vermischt. Zuletzt wird das Magnesiumstearat durch ein Sieb mit einer Maschenweite von 0,8 mm hinzugesiebt und nach 3 Minuten weiteren Mischens je 390 mg der Mischung in Gelatinesteckkapseln der Grösse O (elongated) abgefüllt.

Beispiel 8: Eine 0,2%-ige Injektions- bzw. Infusionslösung kann beispielsweise folgendermassen hergestellt werden.

Wirkstoff, z.B. 3-[N-(5-Phenylpentyl)-N-methyl-amino]-1-hydroxy-propan-1,1-diphosphonsäure oder eines ihrer Salze,

| | |
|---|---|
| z.B. ihr Dinatriumsalz | 5,0 g |
| Natriumchlorid | 22,5 g |
| Phosphatpuffer pH = 7,4 | 300,0 g |
| Wasser, entmin. | ad 2500,0 ml |

Der Wirkstoff wird in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glas- oder Kunststoffampullen (enthaltend je 2,0 bzw. 5,0 mg Wirkstoff abgefüllt.

## Patentansprüche (Patentansprüche für folgende(n) Vertragsstaat(en): AT, BE, CH, DE, FR, GB, IT, LI, LU, NL, SE)

1. Aromatisch substituierte Alkylaminoalkandiphosphonsäuren der Formel 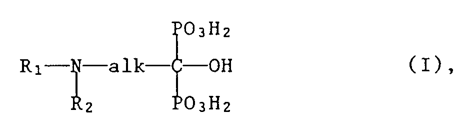 worin R₁ Phenyl-C₄-C₇-alkyl oder Phenyl-C₄-C₇-alkenyl bedeutet, R₂ C₁-C₄-Alkyl oder C₂-G₄-Alkenyl darstellt und alk für C₂-C₄-Alkylen steht, und ihre Salze.

2. Verbindungen gemäss Anspruch 1 der Formel I, worin R₁ geradkettiges Phenyl-C₄-C₇-alkyl bedeutet, R₂ C₁-C₄-Alkyl darstellt und alk für C₂-C₃-Alkylen steht, und ihre Salze.

3. Verbindungen gemäss Anspruch 1 der Formel I, worin R₁ geradkettiges Phenyl-C₄-C₇-alkyl bedeutet, R₂ C₁-C₄-Alkyl darstellt und alk für Aethylen steht, und ihrer Salze.

4. 3-[N-(4-Phenylbutyl)-N-methyl-amino]-1-hydroxy-propan-1,1-diphosphonsäure oder ein Salz davon.

5. 3-[N-(5-Phenylpentyl)-N-methyl-amino]-1-hydroxy-propan-1,1-diphosphonsäure oder ein Salz davon.

6. 3-[N-(6-Phenylhexyl)-N-methyl-amino]-1-hydroxy-propan-1,1-diphosphonsäure oder ein Salz davon.

7. 3-[N-(7-Phenylheptyl)-N-methyl-amino]-1-hydroxy-propan-1,1-diphosphonsäure oder ein Salz davon.

8. Eine Verbindung gemäss einem der Ansprüche 1-7 in freier Form oder in pharmazeutisch verwendbarer Salzform zur Anwendung in einem Verfahren zur therapeutischen Behandlung des menschlichen oder tierischen Körpers.

9. Pharmazeutische Präparate enthaltend eine Verbindung gemäss einem der Ansprüche 1-7 neben üblichen pharmazeutischen Trägerstoffen.

10. Verfahren zur Herstellung aromatisch substituierter Alkylaminoalkandiphosphonsäuren der Formel (I) gemäss Anspruch 1, oder Salzen davon,
dadurch gekennzeichnet, dass man
a) in einer Verbindung der Formel 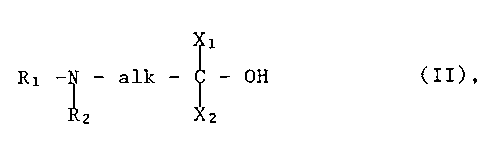 worin X₁ eine funktionell abgewandelte und X₂ eine freie oder funktionell abgewandelte Phosphonogruppe bedeutet, funktionell abgewandeltes Phosphono X₁ und gegebenenfalls X₂ in die freie Phosphonogruppe überführt, oder
b) Verbindungen der Formeln
R_{A} - Y₁ (III)
und 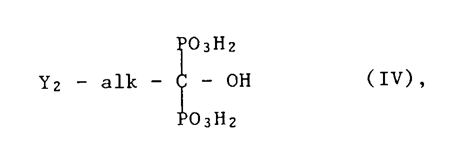 worin einer der Reste Y₁ und Y₂ eine reaktionsfähige veresterte Hydroxygruppe und der andere eine Gruppe der Formel -N(R_{B})-H bedeutet, wobei einer der Reste R_{A} und R_{B} einen Rest R₁ und der andere einen Rest R₂ darstellt, oder deren Salze, miteinander umsetzt, oder unter reduzierenden Bedingungen Verbindungen der Formeln
R_{C}=O (IIIa)
und 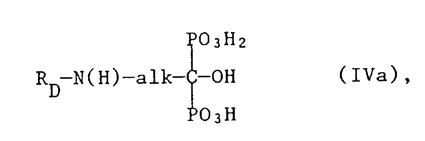 worin R_{C} ein dem Rest R₁ entsprechender doppelt gebundener Rest, z.B. Phenyl-C₄-C₇-alkylidenrest oder Phenyl-C₄-C₇-alkenylidenrest, und R_{D} einen Rest R₂ oder R_{C} einen dem Rest R₂ entsprechender doppelt gebundener Rest, z.B. C₁-C₄-Alkyliden, und R_{D} einen Rest R₁ bedeutet, oder
c) eine Verbindung der Formel 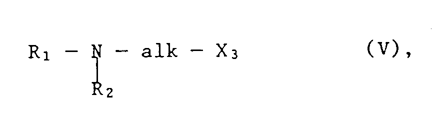 worin X₃ Carboxy, Carbamyl oder Cyano bedeutet, mit einem Phosphorylierungsmittel umsetzt und das Primärprodukt hydrolysiert; und im Falle von X₃=Cyano oder X₃=Carbamyl das erhaltene Zwischenprodukt der Formel 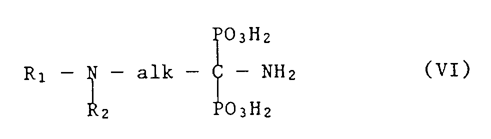 bzw. ein Salz davon mit salpetriger Säure behandelt, und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

11. Verwendung einer Verbindung gemäss einem der Ansprüche 1 bis 7 zur Herstellung eines zur Behandlung von Störungen des Calciumstoffwechsels und damit verbundenen Erkrankungen geeigneten Arzneimittels.

## Patentansprüche (Patentansprüche für folgende(n) Vertragsstaat(en): ES, GR)

1. Verfahren zur Herstellung aromatisch substituierter Alkylaminoalkandiphosphosäuren der Formel 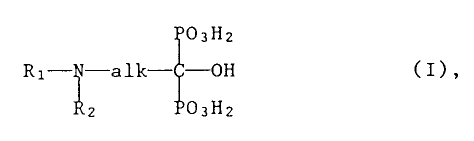 worin R₁ Phenyl-C₄-C₇-alkyl oder Phenyl-C₄-C₇-alkenyl bedeutet, R₂ C₁-C₄-Alkyl oder C₂-C₄-Alkenyl darstellt und alk für C₂-C₄-Alkylen steht, und ihren Salzen,
dadurch gekennzeichnet, dass man
a) in einer Verbindung der Formel 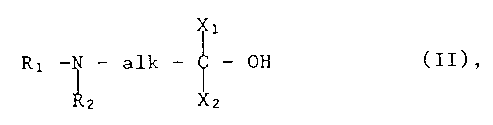 worin X₁ eine funktionell abgewandelte und X₂ eine freie oder funktionell abgewandelte Phosphonogruppe bedeutet, funktionell abgewandeltes Phosphono X₁ und gegebenenfalls X₂ in die freie Phosphonogruppe überführt, oder
b) Verbindungen der Formeln
R_{A} - Y₁ (III)
und 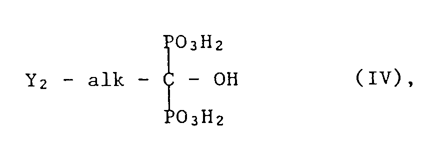 worin einer der Reste Y₁ und Y₂ eine reaktionsfähige veresterte Hydroxygruppe und der andere eine Gruppe der Formel -N(R_{B})-H bedeutet, wobei einer der Reste R_{A} und R_{B} einen Rest R₁ und der andere einen Rest R₂ darstellt, oder deren Salze, miteinander umsetzt, oder unter reduzierenden Bedingungen Verbindungen der Formeln
R_{C}=O (IIIa)
und 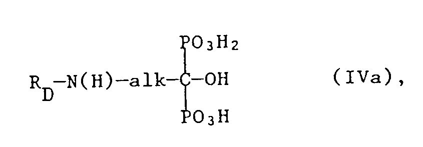 worin R_{C} ein dem Rest R₁ entsprechender doppelt gebundener Rest, z.B. Phenyl-C₄-C₇-alkylidenrest oder Phenyl-C₄-C₇-alkenylidenrest, und R_{D} einen Rest R₂ oder R_{C} einen dem Rest R₂ entsprechender doppelt gebundener Rest, z.B. C₁-C₄-Alkyliden, und R_{D} einen Rest R₁ bedeutet, oder
c) eine Verbindung der Formel 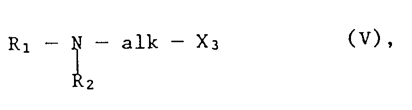 worin X₃ Carboxy, Carbamyl oder Cyano bedeutet, mit einem Phosphorylierungsmittel umsetzt und das Primärprodukt hydrolysiert; und im Falle von X₃=Cyano oder X₃=Carbamyl das erhaltene Zwischenprodukt der Formel 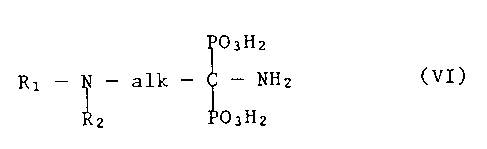 bzw. ein Salz davon mit salpetriger Säure behandelt, und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

2. Verfahren gemäss Anspruch 1 zur Herstellung von Verbindungen der Formel I, worin R₁ geradkettiges Phenyl-C₄-C₇-alkyl bedeutet, R₂ C₁-C₄-Alkyl darstellt und alk für C₂-C₃-Alkylen steht, und ihrer Salze.

3. Verfahren gemäss Anspruch 1 zur Herstellung von Verbindungen der Formel I, worin R₁ geradkettiges Phenyl-C₄-C₇-alkyl bedeutet, R₂ C₁-C₄-Alkyl darstellt und alk für Aethylen steht, und ihrer Salze.

4. Verfahren gemäss Anspruch 1 zur Herstellung von 3-[N-(4-Phenylbutyl)-N-methyl-amino]-1-hydroxy-propan-1,1-diphosphonsäure oder eines Salzes davon.

5. Verfahren gemäss Anspruch 1 zur Herstellung von 3-[N-(5-Phenylpentyl)-N-methyl-amino]-1-hydroxy-propan-1,1-diphosphonsaure oder eines Salzes davon.

6. Verfahren gemäss Anspruch 1 zur Herstellung von 3-[N-(6-Phenylhexyl)-N-methyl-amino]-1-hydroxy-propan-1,1-diphosphonsäure oder eines Salzes davon.

7. Verfahren gemäss Anspruch 1 zur Herstellung von 3-[N-(7-Phenylheptyl)-N-methyl-amino]-1-hydroxy-propan-1,1-diphosphonsäure oder eines Salzes davon.

8. Verfahren zur Herstellung pharmazeutischer Präparate, dadurch gekennzeichnet, dass man eine Verbindung erhältlich gemäss einem der Ansprüche 1-7 mit üblichen pharmazeutischen Trägerstoffen vermischt.

9. Verfahren gemäss Anspruch 8 zur Herstellung eines zur Behandlung von Störungen des Calciumstoffwechsels und damit verbundenen Erkrankungen geeigneten Arzneimittels.

## Claims (Claims for the following Contracting State(s): AT, BE, CH, DE, FR, GB, IT, LI, LU, NL, SE)

1. An alkylaminoalkanediphosphonic acid having an aromatic substituent of the formula 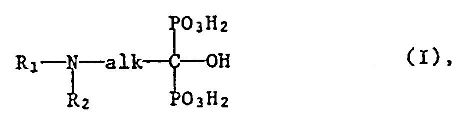 in which R₁ is phenyl-C₄-C₇alkyl or phenylC₄-C₇alkenyl, R₂ is C₁-C₄alkyl or C₂-C₄alkenyl and alk is C₂-C₄alkylene, or a salt thereof.

2. A compound according to claim 1 of the formula I, in which R₁ is straight-chain phenyl-C₄-C₇alkyl, R₂ is C₁-C₄alkyl and alk is C₂-C₃alkylene, or a salt thereof.

3. A compound according to claim 1 of the formula I, in which R₁ is straight-chain phenyl-C₄-C₇alkyl, R₂ is C₁-C₄alkyl and alk is ethylene, or a salt thereof.

4. 3-[N-(4-Phenylbutyl)-N-methylamino]-1-hydroxypropane-1,1-diphosphonic acid or a salt thereof.

5. 3-[N-(5-Phenylpentyl)-N-methylamino]-1-hydroxypropane-1,1-diphosphonic acid or a salt thereof.

6. 3-[N-(6-Phenylhexyl)-N-methylamino]-1-hydroxypropane-1,1-diphosphonic acid or a salt thereof.

7. 3-[N-(7-Phenylheptyl)-N-methylamino]-1-hydroxypropane-1,1-diphosphonic acid or a salt thereof.

8. A compound according to any one of claims 1-7 in the free form or in a pharmaceutically acceptable salt form, for use in a method for the therapeutic treatment of the human or animal body.

9. A pharmaceutical preparation containing a compound according to any one of claims 1-7 in addition to customary pharmaceutical carriers.

10. A process for the preparation of an alkylaminoalkanediphosphonic acid having an aromatic substituent, of formula (I) according to claim 1 or a salt thereof, which comprises
a) converting functionally modified phosphono X₁ and if appropriate X₂ in a compound of the formula 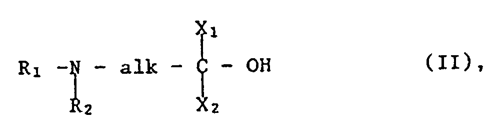 in which X₁ is a functionally modified and X₂ is a free or functionally modified phosphono group, into the free phosphono group, or
b) reacting with one another compounds of the formulae
R_{A} - Y₁ (III)
and 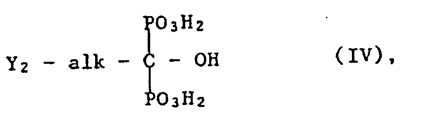 in which one of the radicals Y₁ and Y₂ is a reactive esterified hydroxyl group and the other is a group of the formula -N(R_{B})-H, in which one of the radicals R_{A} and R_{B} is a radical R₁ and the other is a radical R₂, or salts thereof, or reacting compounds of the formulae
R_{C}=O (IIIa)
and 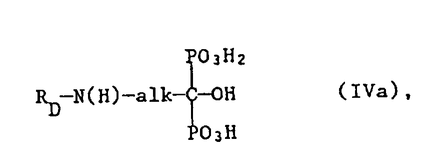 in which R_{C} is a divalent radical corresponding to the radical R₁, for example a phenyl-C₄-C₇alkylidene radical or phenyl-C₄-C₇alkenylidene radical, and R_{D} is a radical R₂, or R_{C} is a divalent radical corresponding to the radical R₂, for example C₁-C₄alkylidene, and R_{D} is a radical R₁, under reducing conditions, or
c) reacting a compound of the formula 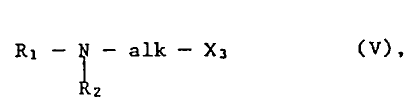 in which X₃ is carboxyl, carbamyl or cyano with a phosphorylating agent and hydrolysing the primary product; and in the case in which X₃ is cyano or X₃ = carbamyl, treating the resulting intermediate of the formula 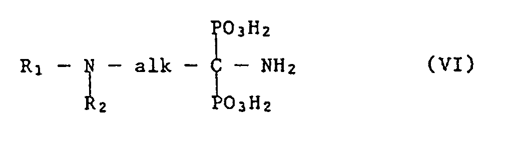 or a salt thereof with nitrous acid, and if desired converting a resulting compound into another compound of the formula I and/or converting a resulting free compound into a salt or a resulting salt into the free compound or into another salt.

11. The use of a compound according to any one of claims 1 to 7 for the preparation of a medicament suitable for the treatment of disturbances in calcium metabolism and associated diseases.

## Claims (Claims for the following Contracting State(s): ES, GR)

1. A process for the preparation of an alkylaminoalkanediphosphonic acid having an aromatic substituent, of the formula 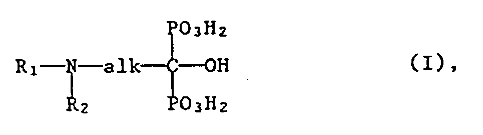 in which R₁ is phenyl-C₄-C₇alkyl or phenyl-C₄-C₇alkenyl, R₂ is C₁-C₄alkyl or C₂-C₄alkenyl and alk is C₂-C₄alkylene, or a salt thereof, which comprises
a) converting functionally modified phosphono X₁ and if appropriate X₂ in a compound of the formula 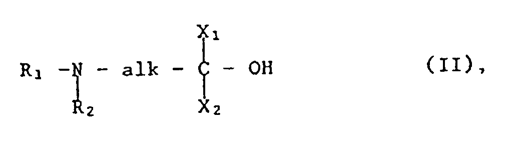 in which X₁ is a functionally modified and X₂ is a free or functionally modified phosphono group, into the free phosphono group, or
b) reacting with one another compounds of the formulae
R_{A} - Y₁ (III)
and 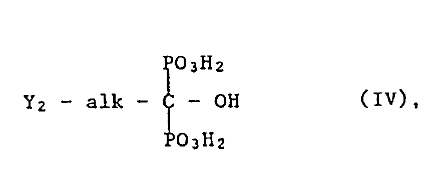 in which one of the radicals Y₁ and Y₂ is a reactive esterified hydroxyl group and the other is a group of the formula -N(R_{B})-H, in which one of the radicals R_{A} and R_{B} is a radical R₁ and the other is a radical R₂, or salts thereof, or reacting compounds of the formulae
R_{C}=O (IIIa)
and 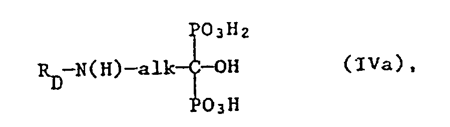 in which R_{C} is a divalent radical corresponding to the radical R₁, for example a phenyl-C₄-C₇alkylidene radical or phenyl-C₄-C₇alkenylidene radical, and R_{D} is a radical R₂, or R_{C} is a divalent radical corresponding to the radical R₂, for example C₁-C₄alkylidene, and R_{D} is a radical R₁, under reducing conditions, or
c) reacting a compound of the formula 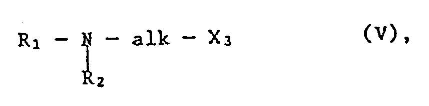 in which X₃ is carboxyl, carbamyl or cyano, with a phosphorylating agent and hydrolysing the primary product; and in the case in which X₃ is cyano or X₃ = carbamyl, treating the resulting intermediate of the formula 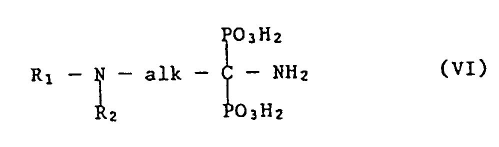 or a salt thereof with nitrous acid, and if desired converting a resulting compound into another compound of the formula I and/or converting a resulting free compound into a salt or a resulting salt into the free compound or into another salt.

2. A process according to claim 1 for the preparation of compounds of the formula I in which R₁ is straight-chain phenyl-C₄-C₇alkyl, R₂ is C₁-C₄alkyl and alk is C₂-C₃alkylene, and a salt thereof.

3. A process according to claim 1 for the preparation of compounds of the formula I in which R₁ is straight-chain phenyl-C₄-C₇alkyl, R₂ is C₁-C₄alkyl and alk is ethylene, and salts thereof.

4. A process according to claim 1 for the preparation of 3-[N-(4-phenylbutyl)-N-methylamino]-1-hydroxypropane-1,1-diphosphonic acid or a salt thereof.

5. A process according to claim 1 for the preparation of 3-[N-(5-phenylpentyl)-N-methylamino]-1-hydroxypropane-1,1-diphosphonic acid or a salt thereof.

6. A process according to claim 1 for the preparation of 3-[N-(6-phenylhexyl)-N-methylamino]-1-hydroxypropane-1,1-diphosphonic acid or a salt thereof.

7. A process according to claim 1 for the preparation of 3-[N-(7-phenylheptyl)-N-methylamino]-1-hydroxypropane-1,1-diphosphonic acid or a salt thereof.

8. A process for the production of pharmaceutical preparations, which comprises mixing a compound obtainable according to any one of claims 1-7 with customary pharmaceutical carriers.

9. A process according to claim 8 for the preparation of a medicament suitable for the treatment of disturbances in calcium metabolism and associated diseases.

## Revendications (Revendications pour l'(les) Etat(s) contractant(s) suivant(s): AT, BE, CH, DE, FR, GB, IT, LI, LU, NL, SE)

1. Acides alkylaminoalcane-diphosphoniques à substituants aromatiques substitués de formule 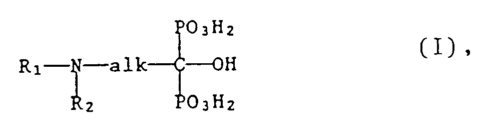 dans laquelle R₁ représente un groupe phényl-alkyle en C 4-C 7 ou phényl-alcényle en C 4-C 7, R₂ représente un groupe alkyle en C 1-C 4 ou alcényle en C 2-C 4 et alk un groupe alkylène en C 2-C 4, et leurs sels.

2. Composés selon revendication 1, de formule I dans laquelle R₁ représente un groupe phényl-alkyle en C 4-C 7 à chaîne droite, R₂ représente un groupe alkyle en C 1-C 4 et alk un groupe alkylène en C 2-C 3, et leurs sels.

3. Composés selon revendication 1, de formule I dans laquelle R₁ représente un groupe phényl-alkyle en C 4-C 7 à chaîne droite, R₂ représente un groupe alkyle en C 1-C 4 et alk le groupe éthylène, et leurs sels.

4. L'acide 3-[N-(4-phénylbutyl)-N-méthylamino]-1-hydroxypropane-1,1-diphosphonique ou l'un de ses sels.

5. L'acide 3-[N-(5-phénylpentyl)-N-méthylamino]-1-hydroxypropane-1,1-diphosphonique ou l'un de ses sels.

6. L'acide 3-[N-(6-phénylhexyl)-N-méthylamino]-1-hydroxypropane-1,1-diphosphonique ou l'un de ses sels.

7. L'acide 3-[N-(7-phénylheptyl)-N-méthylamino]-1-hydroxypropane-1,1-diphosphonique ou l'un de ses sels.

8. Un composé selon l'une des revendications 1 à 7, à l'état libre ou à l'état de sel acceptable pour l'usage pharmaceutique, pour l'utilisation dans un procédé pour le traitement thérapeutique de l'organisme humain ou animal.

9. Compositions pharmaceutiques contenant un composé selon l'une des revendications 1 à 7 avec des véhicules pharmaceutiques usuels.

10. Procédé de préparation des acides alkylaminoalcane-diphosphoniques à substituants aromatiques de formule I de la revendication 1, caractérisé en ce que :
a) dans un composé de formule 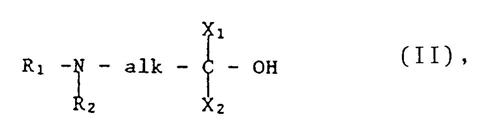 dans laquelle X₁ représente un groupe phosphono ayant subi une modification fonctionnelle et X₂ un groupe phophono libre ou ayant subi une modification fonctionnelle, on convertit en groupes phosphono libres le groupe phosphono ayant subi une modification fonctionnelle X₁ et le cas échéant le groupe X₂, ou bien
b) on fait réagir entre eux des composés de formules
R_{A} - Y₁ (III)
et 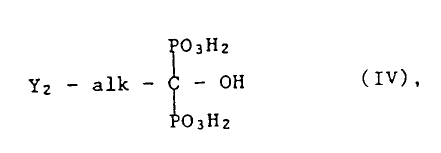 dans laquelle l'un des symboles Y₁ et Y₂ représente un groupe hydroxy estérifié réactif et l'autre un groupe de formule -N(R_{B})-H, l'un des groupes R_{A} et R_{B} est un groupe R₁ et l'autre un groupe R₂, ou leurs sels, ou bien, dans des conditions réductrices, des composés de formules
R_{C}=O (IIIa)
et 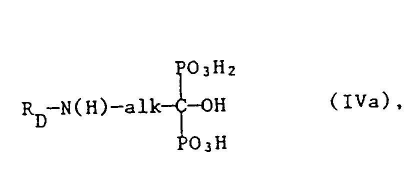 dans laquelle R_{C} est un groupe correspondant au groupe R₁, mais à double liaison, par exemple un groupe phénylalkylidène en C 4-C 7 ou phényl-alcénylidène en C 4-C 7, et R_{D} est un groupe R₂, ou bien R_{C} est un groupe correspondant au groupe R₂ mais à double liaison, par exemple un groupe alkylidène en C 1-C 4, et R_{D} est un groupe R₁, ou bien
c) on fait réagir un composé de formule 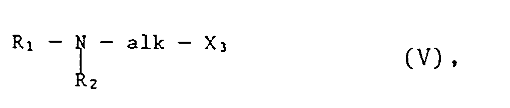 dans laquelle X₃ représente un groupe carboxy, carbamyle ou cyano, avec un agent phosphorylant et on hydrolyse le produit obtenu en premier ; et lorsque X₃ = cyano ou bien X₃ = carbamyle, on traite le produit intermédiaire obtenu, de formule 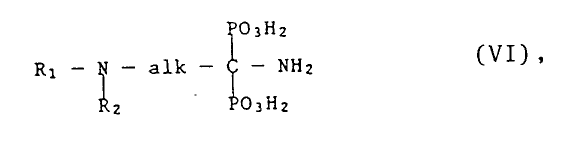 ou l'un de ses sels, par l'acide nitreux, et si on le désire, on convertit un composé ainsi obtenu en un autre composé de formule I et/ou un composé obtenu à l'état libre en un sel ou un composé obtenu à l'état de sel en le composé libre ou en un autre sel.

11. Utilisation d'un composé selon l'une des revendications 1 à 7 pour la préparation d'un médicament prévu pour le traitement des troubles du métabolisme du calcium et des maladies en relation avec ces troubles.

## Revendications (Revendications pour l'(les) Etat(s) contractant(s) suivant(s): ES, GR)

1. Procédé de préparation des acides alkylaminoalcane-diphosphoniques à substituants aromatiques de formule 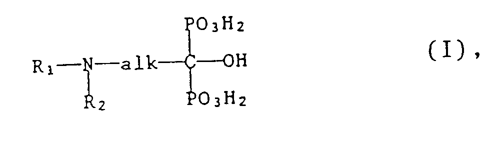 dans laquelle R₁ représente un groupe phényl-alkyle en C 4-C 7 ou phényl-alcényle en C 4-C 7, R₂ un groupe alkyle en C 1-C 4 ou alcényle en C 2-C 4 et alk un groupe alkylène en C 2-C 4, et de leurs sels,
caractérisé en ce que
a) dans un composé de formule 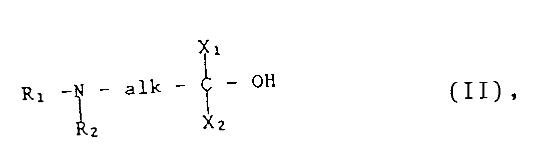 dans laquelle X₁ représente un groupe phosphono ayant subi une modification fonctionnelle et X₂ un groupe phosphono libre ou ayant subi une modification fonctionnelle, on convertit en groupes phosphono libres le groupe phosphono ayant subi une modification fonctionnelle X₁ et le cas échéant le groupe X₂, ou bien
b) on fait réagir entre eux des composés de formules
R_{A} - Y₁ (III)
et 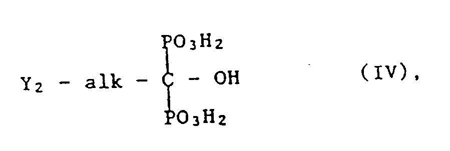 dans laquelle l'un des symboles Y₁ et Y₂ représente un groupe hydroxy estérifié réactif et l'autre un groupe de formule -N(R_{B})-H, l'un des groupes R_{A} et R_{B} est un groupe R₁ et l'autre un groupe R₂, ou leurs sels, ou bien, dans des conditions réductrices, des composés de formules
R_{C}=O (IIIa)
et 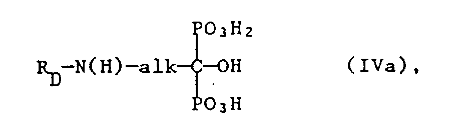 dans laquelle R_{C} est un groupe correspondant au groupe R₁, mais à double liaison, par exemple un groupe phényl-alkylidène en C 4-C 7 ou phényl-alcénylidène en C 4-C 7, et R_{D} est un groupe R₂, ou bien R_{C} est un groupe correspondant au groupe R₂ mais à double liaison, par exemple un groupe alkylidène en C 1-C 4, et R_{D} est un groupe R₁, ou bien
c) on fait réagir un composé de formule 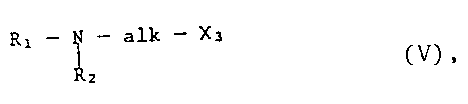 dans laquelle X₃ représente un groupe carboxy, carbamyle ou cyano, avec un agent phosphorylant et on hydrolyse le produit obtenu en premier ; et lorsque X₃ = cyano ou bien X₃ = carbamyle, on traite le produit intermédiaire obtenu, de formule 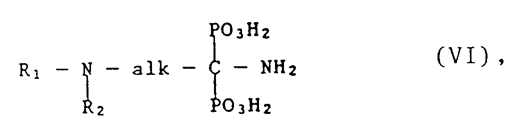 ou l'un de ses sels, par l'acide nitreux, et si on le désire, on convertit un composé ainsi obtenu en un autre composé de formule I et/ou un composé obtenu à l'état libre en un sel ou un composé obtenu à l'état de sel en le composé libre ou en un autre sel.

2. Procédé selon revendication 1, pour la préparation des composés de formule I dans laquelle R₁ représente un groupe phényl-alkyle en C 4-C 7 à chaîne droite, R₂ représente un groupe alkyle en C 1-C 4 et alk un groupe alkylène en C 2-C 3, et de leurs sels.

3. Procédé selon revendication 1, pour la préparation des composés de formule I dans laquelle R₁ représente un groupe phényl-alkyle en C 4-C 7 à chaîne droite, R₂ un groupe alkyle en C 1-C 4 et alk un groupe éthylène, et de leurs sels.

4. Procédé selon revendication 1, pour la préparation de l'acide 3-[N-(4-phénylbutyl)-N-méthylamino]-1-hydroxypropane-1,1-diphosphonique ou l'un de ses sels.

5. Procédé selon revendication 1, pour la préparation de l'acide 3-[N-(5-phénylpentyl)-N-méthylamino]-1-hydroxypropane-1,1-diphosphonique ou l'un de ses sels.

6. Procédé selon revendication 1, pour la préparation de l'acide 3-[N-(6-phénylhexyl)-N-méthylamino]-1-hydroxypropane-1,1-diphosphonique ou l'un de ses sels.

7. Procédé selon revendication 1, pour la préparation de l'acide 3-[N-(7-phénylheptyl)-N-méthylamino]-1-hydroxypropane-1,1-diphosphonique ou l'un de ses sels.

8. Procédé de préparation de compositions pharmaceutiques, caractérisé en ce que l'on mélange un composé obtenu selon l'une des revendications 1 à 7 avec des véhicules pharmaceutiques usuels.

9. Procédé selon revendication 8, pour la préparation d'un médicament convenant pour le traitement des troubles du métabolisme du calcium et des maladies en relation avec ces troubles.
